Europäisches Patentamt

European Patent Office

Office européen des brevets

(11) Publication number: 0 356 774
A1

## (12) EUROPEAN PATENT APPLICATION

(21) Application number: 89114714.2

(22) Date of filing: 09.08.89

(51) Int. Cl.5: A61M 25/00

(30) Priority: 31.08.88 US 238807

(43) Date of publication of application:
07.03.90 Bulletin 90/10

(84) Designated Contracting States:
AT BE CH DE ES FR GB IT LI LU NL SE

(71) Applicant: EDWARD WECK, INC.
200 Headquarters Drive
Skillman New Jersey 08558(US)

(72) Inventor: Davis, Tommy G.
Route 3 - P.O.Box 3311
Athens Texas 75751(US)
Inventor: Hayes, Kelli L.
Route 7 - P.O.Box 7639
Athens Texas 75751(US)

(74) Representative: Modiano, Guido et al
MODIANO, JOSIF, PISANTY & STAUB
Modiano & Associati Baaderstrasse 3
D-8000 München 5(DE)

(54) Catheter.

(57) An improved method of making a catheter (10) such as an over the needle catheter and made therefrom is disclosed. The method comprises forming a headed portion on a length of flexible tubing (22) by pressing a small, metallic, (or plastic) cylindrical insert into the proximal end of the tubing (22) preformed to accept the insert. A hub (24) is injection molded with an internal recess which forms a first internal surface with a narrower through hole in the forward end of the hub (24). A third interior portion of the hub (24) at the rearward end forms a an internal shoulder where it meets the recess. The tubing (22) is passed through the hub (24) so that the headed portion seats itself in the recess against the first internal surface. A heated die or ultrasonically driven staking tool is inserted through the rear end of the hub (24) to press against the internal shoulder to form a narrowed diameter which terminates against the rear end of the insert in the headed portion to thereby lock the tubing (22) in sealing engagement with the hub (24). A smooth, step free transitional portion is formed between the third portion and the recess where the die or tool has a frustum-shaped surface.

FIG. 1

# CATHETER

This invention relates to a method of joining a catheter and its hub and the catheter made thereby.

By way of example, an over the needle catheter consists of two basic components: an inner needle, usually constructed of a stainless steel cannula, beveled to allow penetration, with an injection molded or steel hub; and an outer catheter consisting of a tipped catheter tubing (pvc, urethane, teflon or any biocompatible material), also with an injection molded or steel hub. Hubs for catheters or needles can be of any biocompatible thermoplastic or steel.

OTN catheters utilize a sharp inner needle to introduce the catheter percutaneously. OTN catheters differ from other catheters in that they do not require introducers i.e., dilator, sheath, hemostasis assemblies.

OTN catheters are generally used for supplying fluids to the vascular system, however they are sometimes used to introduce guidewires instead of using a two or three part needle such as a cournand, modified cournand, or percutaneous needle.

Most of the OTN catheters on the market utilize the same inner needle design. The main differences however, are in the catheter construction. All catheters utilize a taper tipped plastic tubing generally thermoformed or formed by some mechanical means. Attachment of the catheter tubing to the hub is accomplished in two ways; mechanical means (heading), or chemical bonding.

One of the most common ways is to thermoform a head on the tubing (a region of increased catheter outside diameter) and encapsulate the head by injection molding around it. This type of molding is often referred to as insert molding.

Another alternative to attaching the thermoformed headed tubing to the hub is to injection mold just the hub leaving a pocket for the head, and heat staking below the head. See U.S. Patent No. 3,469,579.

Stainless steel eyelets or small metal inserts are commonly used to head OTN tubing, by pressing the eyelet or small metal insert into the tubing. This method expands the diameter of the tubing at the proximal end forming a head. Catheters headed by this method are pressed into an injection molded hub or insert molded.

With respect to chemical bonding, if possible, hubs and catheter tubings can be made of homogenous materials. In most cases when homogeneous thermoplastics are insert molded a bonding or fusing together forms. Applications of this type do not require the catheter tubing to be headed.

Plastic inserts are another possibility. For example, polypropylene inserts can be pressed into Teflon tubing. The polypropylene head can then be insert molded into a hub of similar material and a bond will form between the head and the hub.

It is often desirable, however, to use a flexible plastic such as Teflon for the catheter and a relatively rigid plastic such as polypropylene for the hub. Other materials are also desirable such as polyethelene, polyurethane, ABS, or any bio-compatible thermoplastic that can be injection molded and heat staked. Because an effective bonding agent for different plastics such as these is not available, mechanical methods of joining are employed. Less expensive, but effective methods of leak proof, secure mechanical joining of dissimilar plastics for OTN catheters and other medical devices employing a hub and length of flexible tubing such as regular catheters, introducers, sheaths, etc., are always being sought.

A method of making a catheter is provided which includes heading a length of flexible tubing with a small rigid insert by pressing the insert into one end of the tubing. The insert may be made of plastic or metal. Stainless steel is a suitable material for the insert. To accommodate the insert the tubing is preformed using a warm die inserted in an end of the tubing.

A plastic hub is formed, preferably by injection molding. The hub has a hollow interior and is open at both ends. The hollow interior includes a first cylindrical portion, preferably having a diameter just larger than the remaining portion of the tubing outer diameter, and open at the forward end of the hub to ambient atmosphere. The first portion at its opposite end is coaxial with and adjacent to a second cylindrical portion adapted to receive the headed portion of the tubing with the rigid insert. Where the first and second portions meet a first annular surface is formed The hub interior comprises a third portion which opens at the rear end of the hub to ambient atmosphere and which forms an annular ledge where it meets the second interior portion.

The tubing is pressed through the hub, entering from the rear of the hub with the tubing passing through the first interior portion until the headed portion seats in the second interior portion with the forward end of the insert pressing the tubing wall against the first annular surface.

The annular ledge is then formed into a surface having a narrower diameter to form a second annular surface which engages the rearward end surface of the insert, thereby locking the tubing into sealing engagement with the hub. In the preferred embodiment the surface with a narrower diameter is a frustum-shaped surface, but a cylindrical surface could be used.

The narrower diameter surface is formed by inserting a heated staking die through the rear open end of the hub until the die engages the annular ledge. The die is inserted into the hub until the second annular surface is formed engaging the proximal end of the insert. Alternatively, an ultrasonic welding staking die can be used in place of a heated die.

The catheter formed by the above method is also provided. It includes a rigid insert pressed into a proximal end of a length of tubing to form a headed portion which portion is then captured in an interior recess of the hub between a first annular surface, formed when a tubing through hole meets the recess, and a second annular surface formed from a transitional portion of the hub interior which extends between a larger diameter third portion of the interior at the rearward end of the hub adapted to receive the hub of a needle and the recess. In the preferred embodiment, the transition portion has a frustum-shaped interior surface.

FIG. 1 is an enlarged elevation view in partial cross section of an over the needle catheter of the present invention in combination with a needle with which it may be used.

FIG. 2 is an elevation view of a fixture used for joining the hub and tubing portion of the over the needle catheter.

FIG. 3 is a greatly enlarged cross section of the hub and tubing portion before joining them together, along with a first embodiment tool used in joining the hub and tubing portion.

FIG. 4 is a greatly enlarged cross section of the hub and tubing portion of FIG. 3 after joining them together and as shown in FIG. 1.

FIGS. 5A through 5D show the insertion of a rigid insert into an end of the catheter tubing.

FIG. 6 is a greatly enlarged cross section of an alternate embodiment of the hub and tubing portion of FIG. 3 after joining them together.

FIG. 7 is an enlarged alternate embodiment tool used in forming the hub and tubing portion of FIG. 6.

FIG. 8 is an elevation view of an alternate fixture to that of FIG. 2 used for joining the hub and tubing portion of the over the needle catheter.

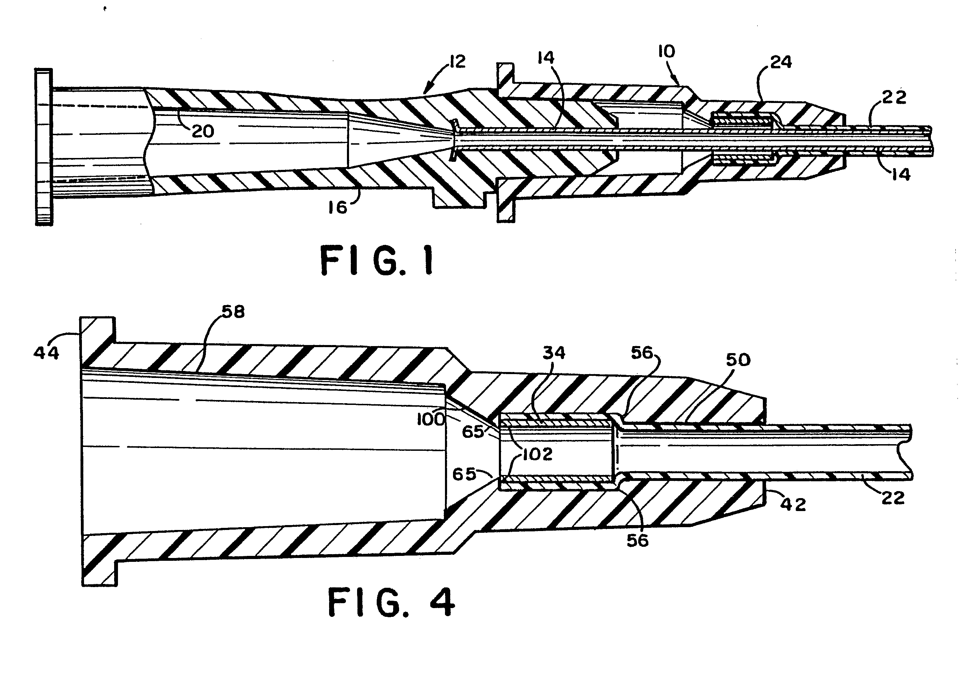

Referring now to FIG. 1, an over the needle catheter designated generally 10 is shown in combination with a needle designated generally 12. The needle comprises an insert molded steel cannula 14 within a thermoplastic hub 16. The steel cannula has a sharpened, beveled end not shown. The hub 16 includes a female luer taper 20 shown schematically and adapted to mate with a standard male luer fitting.

The over the needle catheter comprises a length of flexible tubing 22 and hub 24. The over the needle catheter is then positioned on needle 12 with the cannula 14 extending through a continuous bore in hub 24 and tubing 22. In operation, the needle 12 and over the needle catheter 10 in combination are inserted into the body. The needle 12 is removed from the over the needle catheter with tubing 22 remaining in the body and hub 24 providing means for supplying or withdrawing fluids from the body, etc. From this application it can be seen that it is desirable that the tubing 22 be inert to the body and flexible to conform to the body vessels, while the hub 24 should be more rigid in order to connect to other apparatus.

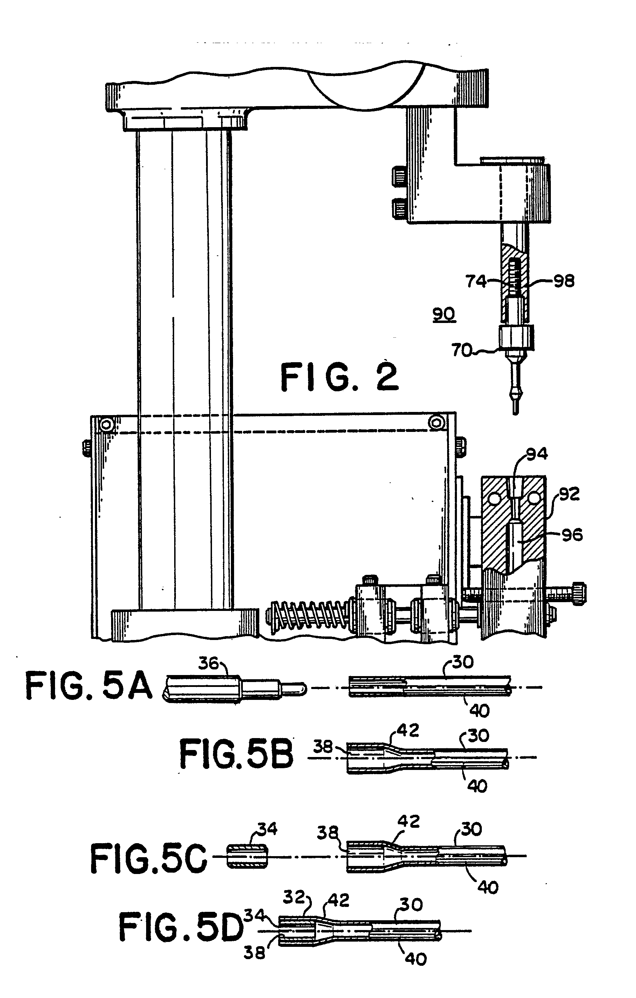

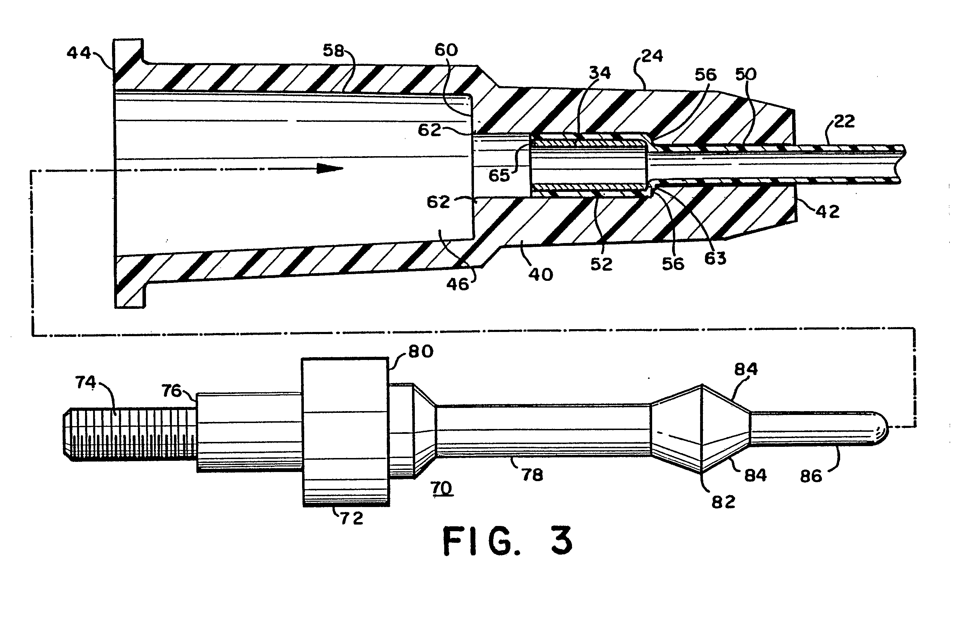

Referring now to FIGS. 5A through 5D, a description of the formation of the tubing 22 is provided. The tubing 22 in its initial form is a length of plastic tubing 30. A suitable and often used material for catheters is tetrapolyfluoroethylene sold under the trademark Teflon®. The plastic tubing 30 is cut to a predetermined length and a headed portion 32 is formed by inserting a rigid cylindrical insert 34 into the proximal end of the tubing. The catheter tubing 30 is first preformed by inserting a warm die 36 into the proximal end. See FIG. 5A. This enlarges the interior of the tubing at its proximal end to form a cylindrical region 38 of larger interior diameter than the remainder 40 of the tubing. Since the interior diameter of the tubing is enlarged so too is the outer diameter in this region. After the warm die is withdrawn from the tubing 30 (FIG. 5B) a short tapered transitional zone 42 is created in the catheter wall between the cylindrical region 38 and the remainder 40 of the tubing.

The rigid cylindrical insert 34 is then pressed into the preformed tubing in the enlarged cylindrical region 38. See FIGS. 5C and 5D. In the preferred embodiment the insert is stainless steel but it could be made of rigid plastic. When using rigid inserts the length of tubing required to form a catheter with a headed portion is predictable and this creates a better product with less waste. More waste is created when headed portions are formed in catheters thermally.

Referring now to FIG. 3, the hub 24 is shown in cross section before permanent joining to the tubing 22. It comprises the hub body 40 having a forward end 42 and a rearward end 44. It further comprises interior passageway 46 which runs the hub's entire length and is open to ambient atmosphere at both the forward and rearward ends. In the preferred embodiment, the hub is plastic and formed by injection molding.

The interior passageway 46 comprises a first cylindrical portion 50 which has a substantially constant interior diameter slightly larger than the outside diameter of the remainder of the tubing portion. Adjacent the first cylindrical portion 50 and coaxial therewith is the second cylindrical portion 52 which has a substantially constant interior diameter. Its interior diameter is slightly larger than the headed portion 32 of the tubing, i.e., than the diameter of the rigid insert 34 and tubing walls within which the insert is located. Where the first and second cylindrical interior portions 50 and 52, respectively, of the passageway meet a first annular surface 56 is formed. The surface 56 is substantially perpendicular to the axis of the first and second portions. The length of the second cylindrical portion 52 is greater than the length of the headed tubing portion 32 for reasons to be explained hereinafter.

The interior passageway further includes a third cylindrical portion 58 which is adjacent to the second cylindrical portion 52 and coaxial with both the first and second portions. The diameter of the third cylindrical portion is a maximum at the rearward end 44 of the hub but decreases linearly along its axis towards the forward end. Even at its smallest the diameter of the third portion is much larger than the diameter of the second and first portions. The decreasing diameter provides a standard female taper for a luer fitting. Where the second and third interior portions of the passageway meet an annular ledge 60 is formed with an interior periphery 62. The surface of the ledge 60 is perpendicular to the axis of the hub.

As shown in FIG. 3, the tubing is passed through the hub and the headed portion 32 comes to rest within the second interior portion 52 against the first annular surface 56. The forward annular end surface 63 of the rigid insert 34 in the headed portion 32 squeezes the tubing wall in the region of the transitional portion 42 against the first annular surface 56. The remainder of the tubing passes through the first cylindrical portion 50 beyond the open forward end 42 of the hub.

Figure 3 further shows a die designated generally 70 for use in staking the headed catheter portion 32 within the hub 24. The die comprises a main cylindrical body 72 with an elongated threaded cylindrical portion 74 extending from one side 76 of the body 72 and a second elongated cylindrical portion 78 extending from an opposite side 80 of the body 72 and coaxial with the portion 74. Attached at the end of the elongated cylindrical portion 78, and preferably machined integrally therewith is an enlarged diameter portion 82, i.e., a portion with a maximum diameter greater than the the diameter of the portion 78. The portion 82 comprises a frustum-shaped distal surface 84 coaxial with the elongated cylindrical portion 78 and extending away from the maximum diameter of the portion 82 and the main body 72. Attached to the end of the frustum-shaped surface 84, and preferably machined integrally therewith, is a terminal elongated cylindrical portion 86 coaxial with the frustum-shaped surface 84.

The diameter of the terminal portion 86 is the same as the diameter of the interior of the rigid insert and tubing. The minimum diameter of the frustum-shaped surface 84 where it meets the terminal portion 86 is the same as the diameter of the terminal portion while its maximum diameter is much larger than the diameter of the second interior portion. The angle of the frustum-shaped surface in the preferred embodiment is thirty degrees measured from the axis of the frustum-shaped surface, but other angles are also suitable depending on the geometry of the components.

Referring now to FIG. 2, a heat staking fixture designated generally 90 is shown for heat staking the headed portion 32 of the tubing within the hub 24 using the die 70. The fixture 90 comprises a catheter hub nesting portion 92 which includes a cavity to accept the hub and catheter of FIG. 3. The hub rests vertically in the cavity portion 94 while the tubing passes through cavity portion 96 below portion 94. The hub and tubing are held in place by gravity.

The elongated cylindrical portion 74 of die 70 seats vertically in a complimentary cavity within die holding portion 98 and the die is locked in place therein vertically above the hub and tubing. The die holding portion 98 and hub nesting portion 92 are coaxially aligned so that when the die holding portion 98 with die 70 is lowered the die will enter through the open rearward end 44 of the hub 24. As the die 70 is lowered farther the terminal portion 86 enters the interior of the rigid insert 34 and the heated frustum-shaped surface 84 contacts the periphery 62 of the the annular ledge 60. As the die is lowered still farther it continually reshapes the periphery 62 of the second annular ledge 60 into a mating frustum-shaped surface until the bottom of the frustum-shaped surface 84 (where it meets the terminal portion 86) contacts the rearward end surface 65 of the rigid insert 32. The die 70 is then withdrawn.

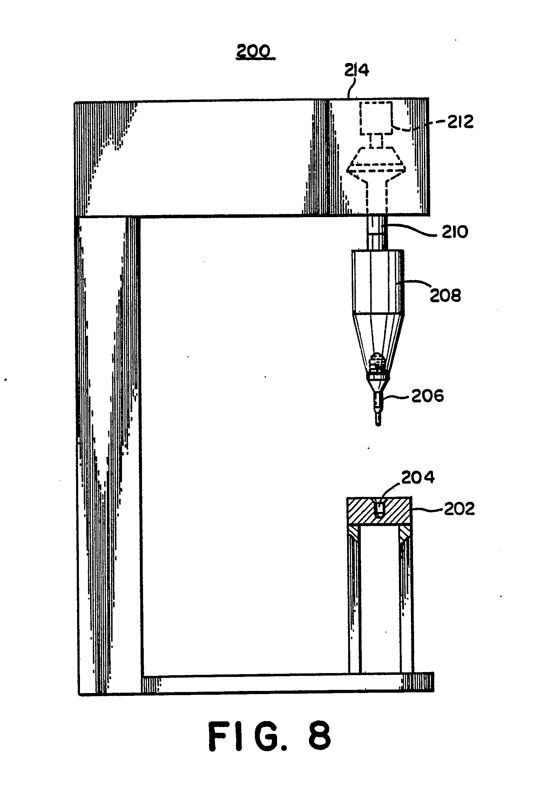

Referring to FIG. 8, a fixture designated generally 200 for ultrasonically welding the headed portion 32 of the catheter within the hub 24 is shown as an alternative to heat staking. The fixture 200 comprises a catheter hub holding portion 202 which includes a cavity 204 to accept the hub 24 while allowing the cathter to pass through and hang below the cavity 204.

An ultrasonic welding staking die 206 is adapted to be held (by mechanical threads or the like) within a welding horn 208 which in turn is coupled to a booster 210. The booster is coupled to a transducer 212. The transducer, booster and horn are supported above the portion 202 by the support 214. The ultrasonic welding equipment includes a power supply not shown which converts 60 Hg electrical energy to 20 kHg. The transducer connects the electrical energy into mechanical energy in the form of longitudinal vibrations and the booster amplifies the vibrations. The horn, which further amplifies the vibrator, holds the die and transmits the vibrator to the parts. A suitable ultrasonic welder is made by Branson Sonic Power Co. of Eagle Road in Danbury, Conneticut.

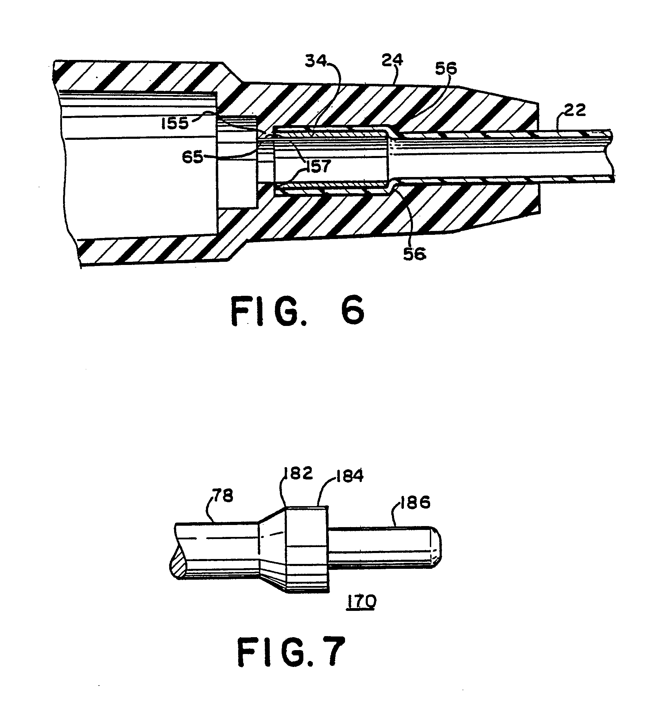

Either of the dies shown in FIGS. 3 or 7 would be suitable for use with the apparatus 200 with modifications which might be necessarty to hold the die within the horn 208. As the longitudinally vibrating die 206 in the form of FIG. 3 is lowered into the open rearward end 44 of the hub 24 the frustum-shaped surface 84 contacts the periphery 62 of the annular ledge 60. As the die is lowered still farther, it continually reshapes the periphery 62 of the second annular ledge 60 into a mating frustum-shaped surface 84 contacts the rearward end surface 65 of the rigid insert 32. The die 70 is then withdrawn.

Referring now to FIG. 4, after the die is withdrawn, the annular ledge 60 has been reshaped to form a frustum-shaped surface 100 which terminates against the rearward end surface 65 of the rigid insert 34 to capture the headed portion 32 of the tubing between the narrowed periphery of frustum-shaped surface 100 and the first annular surface 56. The end of the narrowed periphery forms a second annular surface 102. There is no path for fluid except through the tubing lumen and interior of the rigid insert which together form a lumen from the interior of the third interior portion 58 to the distal open end of the tubing. The tubing is now locked in sealing engagement with the hub.

The process of joining the hub and tubing is inexpensive and secure, and minimizes waste in the quantity of catheter tubing used since there is less uncertainty in how much tubing will be used in forming the headed portion.

A frustum-shaped transitional surface used to capture the headed portion of the tubing within the hub 24 forms a smooth, step free surface for inserting or passing a guidewire through the hub into the catheter tubing without the guidewire snagging or hanging on internal discontinuities at the heat staked region.

It is not necessary, however, to use a frustum-shaped surface to capture the insert. Referring to FIGS. 6 and 7, a second embodiment of a hub and tubing joined together by heat staking or ultrasonic welding is shown. Portions of the hub, tubing and die of FIGS. 6 and 7 are the same as that shown in FIGS. 3 and 4 are referred to with the same numerals in all figures. Starting with a molded hub 24 and headed tubing 22 as shown in FIG. 3, the tool or die 170 of FIG. 7 is inserted into the open end 44 of hub 24. The die 170 is very similar to the die 70 of FIG. 3, except that the portion 182 attached to the end of the elongated cylindrical portion 78 comprises a right circular, cylindrically-shaped distal portion 184 coaxial with the elongated cylindrical portion 78. Similar to the die 70, the portion 184 extends along the die 170 away from the maximum diameter of portion 182 and the main body 72 of the die 170. Attached to the end of the cylindrically-shaped portion 184, and preferably machined integrally therewith, is a terminal elongated cylindrical portion 186 coaxial with the cylindrically-shaped portion 184.

The hub and tubing of FIG. 3 is placed in the catheter hub nesting portion 92 of the fixture 90 (or the cavity 204 of catheter hub holding portion 202) and the die 170 is locked in the die holding portion 98 (or horn 208). As the die 170 is lowered, the die enters the open rearward end 44 of the hub 24 and continues as the terminal portion 186 enters the interior of the rigid insert 34 and the heated cylindrically-shaped portion 184 (or ultrasonically vibrated portion 184) contacts the inner peripheral region 62 of the annular ledge 60. As the die 170 is lowered still farther it continually reshapes the peripheral region 62 of the annular ledge 60 into an annular ring 155 of narrower interior diameter than the initial annular ledge 60 of FIG. 3. The annular ring 155 forms a second annular surface 157 which contacts the rearward end surface 65 of the rigid insert 32. The die is then withdrawn. The headed portion 32 is captured between the first and second annular surfaces 56 and 157, respectively.

The method described herein is not only suitable for over the needle catheters but for the joining of the length of a relatively flexible tube to a more rigid hub to form standard catheters, introducers, sheaths, etc.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. Catheter comprising: a length of flexible tubing having proximal and distal ends and having a hollow rigid cylindrical insert pressed into the proximal end thereof, said insert having an outer diameter substantially larger than the inside diameter of the remainder of the tubing, said insert within said tubing forming a headed portion of said tubing, said tubing and insert forming a passage therethrough, a hub having an interior passage therethrough and forward and rearward ends, said hub passage comprising: a first cylindrical portion at the forward end of said hub; a second cylindrical portion adjacent to and coaxial with the first portion and having a diameter larger than the diameter of said first portion and adapted to receive the headed portion of said tubing, said first and second portions forming a first annular surface where they meet; a third portion having a diameter larger than said second portion throughout its length to receive the hub of a needle; and a transitional portion located between said third portion and said second portion, said transitional portion being narrowed to form a second annular surface of narrowed diameter at said second portion; the walls of said tubing at the forward end of said insert being pressed between said first annular surface and said insert forward end and said second annular surface of said narrower diameter engaging the rearward end of said insert, thereby locking said tubing in sealing engagement with said hub, the interior of said third portion being in communication with the interior of said tubing through the narrowed diameter transitional portion.

2. The catheter of Claim 1 wherein said transitional portion is tapered to from a frustum-shaped surface with said second annular surface of narrowed diameter.

3. The catheter of Claim 2 wherein said hollow rigid insert comprises stainless steel.

4. The catheter of Claim 2 wherein said hollow rigid insert comprises rigid plastic.

5. The catheter of Claim 1 wherein said tubing is formed of tetrapolyfluoroethylene and the hub is formed of polypropylene.

6. The catheter of Claim 1 wherein said tubing is formed of polyurethane and the hub is formed of a thermoplastic.

7. The catheter of Claims 1 through 6 wherein said catheter is an over the needle catheter.

8. A method of making a catheter comprising: inserting a hollow rigid insert of predetermined length in the proximal end of a length of flexible tubing, said insert having an outside diameter greater than the inside diameter of said tubing thereby forming a headed portion on said tubing; forming a plastic catheter hub having a passageway therethrough, said passageway comprising: a first cylindrical portion at the forward end of the hub; a second cylindrical portion adjacent to and coaxial with the first portion having a length greater than said insert predetermined length and a diameter larger than said first portion and slightly larger than said insert and forming an annular surface where the first and second portions meet; and a third portion coaxial with said second portion having a diameter greater than said second portion throughout its length and forming an annular shoulder where the third and second portions meet; pressing the tubing into the hub through the third portion open end to seat the headed portion within the second portion against the first annular surface; and forming a narrowed diameter second annular surface engaging the rearward end of said insert from said annular shoulder thereby locking said tubing in sealing engagement with said hub.

9. The method of Claim 8 wherein the step of forming a narrowed diameter annular surface comprises forming a smooth, step free transitional surface from said annular ledge to from said narrowed diameter annular surface.

10. The method of Claim 9 wherein said step of forming a smooth, step free surface comprises forming a frustum-shaped surface.

11. The method of Claim 8 wherein said step of inserting said insert comprises the step of: inserting a warm die in the proximal end of said tubing to preform the proximal end to receive said insert.

12. The method of Claim 8 wherein said hub forming step comprises injection molding said hub.

13. The method of Claim 11 wherein said hub forming step comprises injection molding said hub.

14. The method of Claim 10 wherein said step of forming a frustum-shaped surface comprises pressing a warm die having a frustum-shaped surface through the open end of said third portion to engage said annular ledge.

15. The method of Claim 10 wherein said step of forming a frustum-shaped surface comprises inserting an ultrasonically driven staking tool having a frustum-shaped portion to engage said annular ledge.

16. The method of Claim 8 wherein said step of forming a narrowed diameter second annular surface comprises pressing a warm die having a right circular cylindrically shaped portion having a diameter smaller than the diameter of said second portion through the open end of said third portion to engage said annular ledge.

17. The method of Claim 8 wherein said step of forming a narrowed diameter second annular surface comprises inserting an ultrasonically driven staking tool having a right annular cylindrically shaped portion having a diameter small than the diameter of said second portion through the open end of said third portion to engage said annular ledge.

FIG. 1

FIG. 4

EP 0 356 774 A1

FIG. 2

FIG. 5A

FIG.5B

FIG.5C

FIG.5D

FIG. 3

FIG. 6

FIG. 7

200

214

212

210

208

206

204

202

FIG. 8

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl.4) |
| X | CH - A - 508400 (VIGGO AB)  * Totality * | 1,3,8, 16 | A 61 M 25/00 |
| Y | | 5-7,12 | |
| A | | 2,9,11 | |
| | -- | | |
| D,Y | US - A - 3 469 579 (F.H. HUBERT)  * Totality; especially column 1, lines 45-47; column 3, lines 26-33 * | 5,7 | |
| | -- | | |
| Y | EP - A1 - 0 227 230 (MENLO CARE) | 6,7 | |
| A | * Fig. 1,3a-c; page 9, lines 18-23; page 10, lines 9-12; page 17, line 28 - page 18, line 32 * | 1 | |
| | -- | | |
| Y | US - A - 4 191 185 (F. LEMIEUX) | 7,12 | TECHNICAL FIELDS SEARCHED (Int. Cl.4) |
| A | * Totality; especially fig. 3; column 4, lines 28-37 * | 1 | A 61 M 5/00  A 61 M 25/00 |
| | -- | | |
| A | US - A - 3 454 006 (A.J. LANGDON)  * Totality; especially fig. 5; column 3, line 46 - column 4, line 24; column 5, lines 31- 36 * | 1,7 | |
| | ---- | | |

The present search report has been drawn up for all claims

| Place of search | Date of completion of the search | Examiner |
|---|---|---|
| VIENNA | 20-12-1989 | LUDWIG |